## Europäisches Patentamt
## European Patent Office
## Office européen des brevets

(11) Publication number: **0 129 985**
**B1**

(12) **EUROPEAN PATENT SPECIFICATION**

(45) Date of publication of patent specification: **07.09.88**

(51) Int. Cl.⁴: **A 61 M 15/00**

(21) Application number: **84303490.1**

(22) Date of filing: **23.05.84**

(54) Inhalation device.

(30) Priority: **24.05.83 GB 8314308**

(43) Date of publication of application:
**02.01.85 Bulletin 85/01**

(45) Publication of the grant of the patent:
**07.09.88 Bulletin 88/36**

(84) Designated Contracting States:
**AT BE CH DE FR IT LI LU NL SE**

(56) References cited:
**EP-A-0 041 783**
**DE-A-2 654 018**
**DE-A-3 016 127**
**US-A-3 425 414**

(73) Proprietor: **GLAXO GROUP LIMITED**
**Clarges House 6-12 Clarges Street**
**London W1Y 8DH (GB)**

(72) Inventor: **Newell, Robert Edward**
**21 The Glen (Off Village Way) Pinner**
**Middlesex HA5 5AX (GB)**
Inventor: **Fitzsimmons, Robert Alexander**
**The Old Vicarage Egglestone**
**Barnard Castle County Durham (GB)**
Inventor: **Price, Philip Thomas**
**4 Brantwood Avenue Isleworth**
**Middlesex (GB)**

(74) Representative: **Boon, Graham Anthony et al .**
**Elkington and Fife High Holborn House 52/54**
**High Holborn**
**London, WC1V 6SH (GB)**

Courier Press, Leamington Spa, England.

**Description**

This invention relates to medical administration devices, particularly devices by which a medicament can be administered to patients inhaling through the devices. Devices used for administering medicaments by inhalation are now well known for administering medicaments to patients suffering from bronchial conditions such as, for example, bronchial asthma. In such known devices, medicament in powder or other finely divided form is supplied in capsules which are loaded by a patient into the inhalation device. The medicament is then released from the capsule and inhaled by the patient usually through the mouth, but sometimes through the nose.

EP—A—0041783 provides a medical inhalation device comprising a body with a chamber therein, the said chamber being adapted to receive a container for medicament; and having a partition on which the container can be supported; a cover which normally closes the chamber but which may be opened to permit the container to be inserted and located in the chamber; a mouthpiece communicating with the chamber and through which a patient may inhale; at least one air inlet leading into the chamber; and opening means engageable with the container, while it is stationary on the partition, when the cover is closed, to open the container supported on the partition, so as to permit medicament to be withdrawn from the container when the patient inhales. It is to be noted that the container is in the form of a capsule and once it has been opened and the patient inhales, the capsule is drawn into a swirl chamber where the air stream therein causes the capsule to spin and thereby expel its powder into the air stream.

An object of the present invention is to provide a more convenient way of administering medicaments to such patients. The device of the present invention makes use of the technique of packing medicaments by encapsulating them in so-called blister packs, that is to say packs comprising a sheet of material which acts as a carrier and which is provided with a number of breakable or openable containers called "blisters" incorporating a sheet secured on a first sheet to form a cover or lid. Such blister packs are in common use with tablets of one kind or another. The present invention provides a way of administering medicaments in finely divided form or liquid form.

According to the present invention there is provided a device of the type described in EP—A—0041783 characterized in that the said container is a blister of a blister pack which is supported on the partition so as to remain stationary not only during opening of the blister but also during inhalation, the partition having a locating aperture extending through it so that the blister may be located therein; the cover is hingedly connected with the body; and the said opening means comprises an opener member carried by the hinged cover and so positioned that when the cover is closed the member can pass through the locating aperture in the partition thereby to open the blister located therein.

The mouthpiece is preferably arranged to be received by the mouth, but can, if desired, be arranged to enter a nostril of the patient to dispense suitable medicaments.

Some embodiments of the invention are illustrated in the accompanying schematic drawings in which:

Figure 1 is a perspective view of a single dose medical inhalation device according to this invention;

Figure 2 is a perspective view of an opening spike forming part of the device;

Figure 3' is a perspective view of a multidose inhaler;

Figure 4 is a detailed view of part of the device;

Figure 5 is a detailed perspective view of another part of the device;

Figure 6 is a perspective view of an alternative construction, and

F igures 7 and 8 are perspective views of another alternative construction.

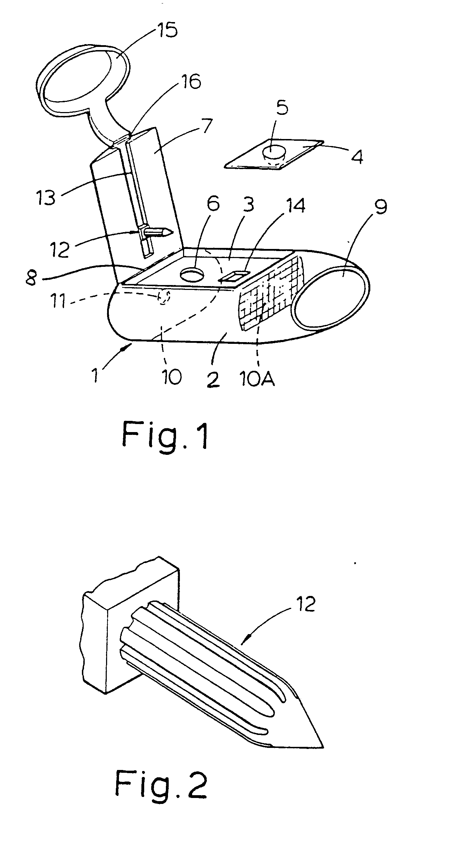

In the embodiment of the invention illustrated in Figures 1 and 2, a medical inhalation device comprises an elongated body 1 of plastics material. This body contains a chamber 2 with an intermediate partition or support 3 arranged to support a blister pack 4 provided with a single blister 5 for medicament in solid finely divided form. The support 3 has a location hole 6 in which the blister 5 of the blister pack can be located.

The body has a lid or cover 7 which may be formed as an integral part of the moulding of the device. The lid or cover 7 being connected with the body 1 by an integral plastics hinge 8 which may be a hinge of the kind commonly known as a living or natural hinge. The body has a mouthpiece 9, the mouthpiece end of the device being herein considered to be the front end. The body has an air inlet 11 which may be in the rear wall 10 and through which air can enter the chamber 2. The front wall 10A of the chamber 2 is in the form of a grid. Inside the cover or lid 7 is an opening spike 12 illustrated in detail in Figure 2. The spike 12 is arranged so that when it is registered with the location hole 6 and the lid 7 is closed the spike will pierce the blister 5 of the blister pack 4 and enable the medicament in the blister pack to enter the chamber 2. The inside of the lid is provided with a track 13 in which the spike 12 can slide. When the device is not in use the spike 12 can be positioned in its track 13 so that when the lid is closed the spike will enter a rest or dwell hole 14 in the partition or support 3. Any suitable means such as grooves (not shown) may be provided for locating the spike in either of these positions.

In use, therefore, the device may be pre-loaded with a blister pack 4 and carried by the patient with the spike 12 located in the hole 14 until he needs to inhale medicament. When he needs to inhale medicament, the patient opens the lid 7 and moves the spike 12 into a position such that when the lid is again closed it will pierce the blister 5 of the blister pack. When the patient then inhales through the mouthpiece 9 the powder will be withdrawn from the blister 5 and the chamber 2 into the patient. The lid is provided with a cap 15 connected to the lid by an integral plastics hinge 16. When the lid is closed the cap 15 may be arranged to fit over the mouthpiece 9.

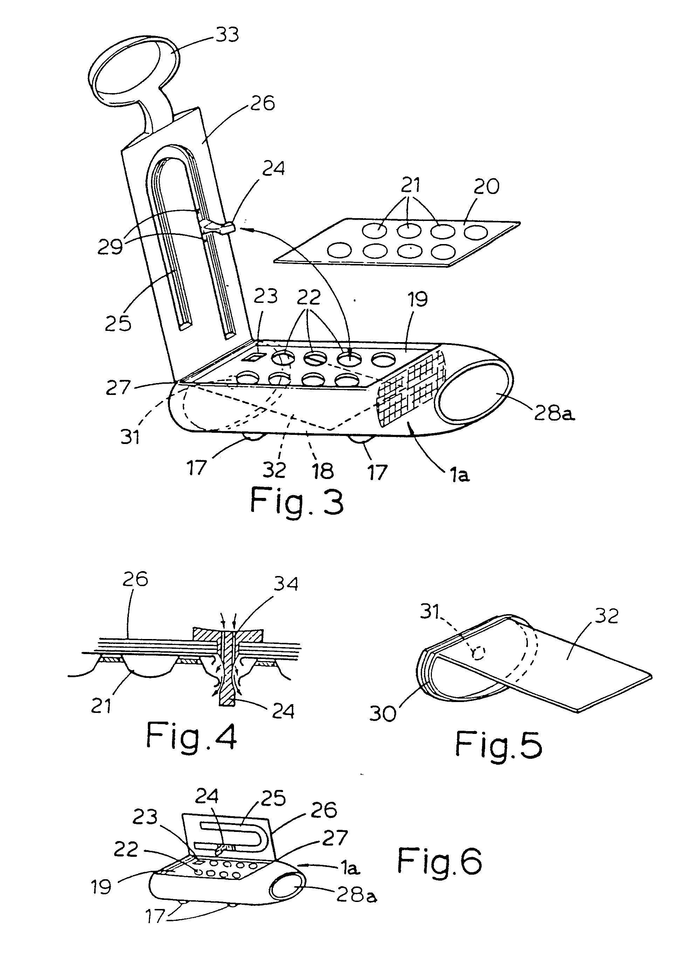

The arrangements illustrated in Figures 3 to 6 inclusive are of generally similar construction but are arranged to provide a multidose device. In the arrangement illustrated in Figure 3 an elongated body 1a is provided with feet 17 to prevent the device from rolling when it is placed on a flat surface. The body 1a includes a chamber 18 with a partition or support 19 for a blister pack 20 having a plurality of blisters 21 for medicament. The partition or support 19 is provided with a plurality of location holes 22 arranged in two rows to correspond with the two rows of blisters 21 in the blister packs 20. The partition 19 also has a hole 23 which is a rest or dwell hole for a spike 24 movable in a track 25 inside a lid 26 which is an integral part of the moulding forming the device and which is connected to the body by an integral plastics hinge 27. A pluralty of grooves 29 or other location means are provided (only two of which are shown in Figure 3) for locating the spike in rgeister with one of the holes 22 or 23. The body is provided with a mouthpiece 28a at its front end. The rear end of the device is provided with a back panel 30 illustrated in Figure 5, this back panel being provided with air inlet holes 31. A baffle plate 32 forms part of the panel 30. A cap 33 for the mouthpiece is connected to the lid 26. In use, a blister pack 20 is loaded into the chamber 18 above the partition or support 19 with the blisters 21 located in the various holes 22. When a patient desires to inhale, the lid is opened and the spike 24 is arranged in a position at which it will when the lid is re-closed register with the appropriate blister 21 and pierce that blister. For convenience of use, the various location holes in the partition 19 may be numbered and similar numbers may be provided adjacent the track 25 to enable the spike to be easily located in the desired position. The spike has grooved or fluted walls, the grooves or flutes 34 (Figure 4) permitting air to enter when the lid is closed. When the lid is closed the spike 24 will pierce through a blister as clearly shown in Figure 4.

When a blister has been pierced the patient may inhale through the mouthpiece and in so doing powder will be entrained in the air being inhaled and will be withdrawn through the mouthpiece.

If desired, there may be only a single row of blisters 21 in the blister pack.

Figure 6 illustrates an alternative in which the hinge 27 is provided at the side of the body 1a instead of at an end.

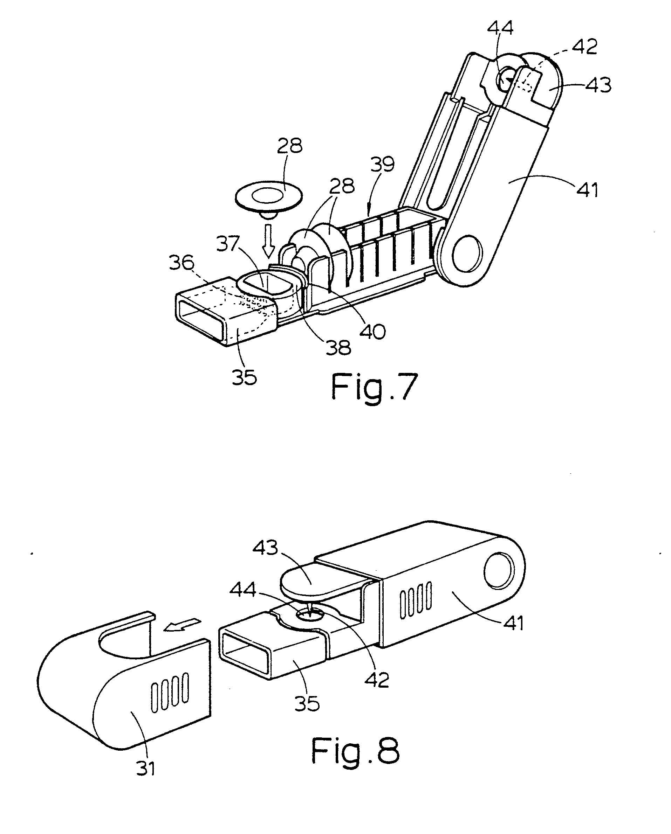

Another modified device is illustrated in Figure 7.

This modification is intended for use with single blister packs 28 each containing one dose of medicament. The device illustrated comprises a body with a mouthpiece portion 35 at one end which is considered to be the front end of the device. A grid 36 is provided at the rear end of the mouthpiece portion. Behind the grid 36 is a chamber 37 with a top or partition 38 having a central aperture. The top 38 supports a blister pack 28.

Behind the chamber 37 is a magazine 39 for spare or unused blister packs 28 such magazine grooves being open at the top and are adapted to contain one or more blister packs 28. An upwardly projecting wall 40 is provided between the magazine and the blister chamber 37.

A lid 41 is hinged at the rear end of the body. When closed, the lid 41 embraces all but the mouthpiece portion of the body and closes the blister chamber 37 and the magazine 39.

A forward portion of the lid 41 has a spike 42, supported by a flexible trigger plate 43. This spike may be provided with air inlet passages similar to those described with reference to Figure 4 of the drawings. If a blister pack 28 is loaded on the support partition 38 the lid is closed and the trigger plate 43 is depressed. The spike 42 will enter the chamber 37 through a hole 44 and pierce through the blister. The patient may then withdraw medicament from the blister into his mouth by inhaling through the mouthpiece.

If desired a removable cover 31 may be fitted on the mouthpiece 35.

The blisters are preferably arranged to contain medicament in solid divided form but they can be arranged to contain a liquid.

**Claims**

1. A medical inhalation device comprising a body (1, 1a) with a chamber (2, 18, 37) therein, the said chamber being adapted to receive a container (5, 21) for medicament and having a partition (3, 19, 38) on which the container can be supported; a cover (7, 26, 41) which normally closes the chamber but which may be opened to permit the container to be inserted and located in the chamber; a mouthpiece (9, 28a, 35) communicating with the chamber and through which a patient may inhale; at least one air inlet (34, 44) leading into the chamber; and opening means (12, 24, 42) engageable with the container, while it is stationary on the partition, when the cover is closed, to open the container supported on the partition, so as to permit medicament to be withdrawn from the container when the patient inhales, characterized in that the said container is a blister (5, 21) of a blister pack (4, 20, 28) which is supported on the partition (3, 19, 38) so as to remain stationary not only during opening of the blister but also during inhalation, the partition having a locating aperture (6, 22) extending through it so that the blister may be located therein; the cover is hingedly connected with the body; and the said opening means comprises an opener member (12, 24, 42) carried by the hinged cover and so positioned that when the cover is closed the member can pass through the locating aperture (6, 22) in the partition thereby to open the blister located therein.

2. A device as claimed in claim 1, wherein the opener member (12, 24) is slidable in a track (13, 25) on the inside of the cover so that the position of the member may be adjusted; and the partition has a dwell aperture (14, 23) in which the member may dwell when the cover is closed without opening a blister located in the locating aperture (6, 22).

3. A device as claimed in claim 1 or claim 2, wherein the cover (7, 26, 41) is arranged when closed to lie adjacent one side of the partition (3, 19, 38) and the said air inlet (34, 44) leads into the chamber on the other side of the partition.

4. A device as claimed in claim 1, wherein the partition (19) is adapted to support a pack (20) provided with a plurality of blisters (21) and has a plurality of locating apertures (22) each of which is arranged to receive one of the blisters, the opener member (24) being movable in a track (25) inside the cover so that its position may be adjusted to register with that of any one of the locating apertures (22) or with that of a dwell aperture (23) in the partition (19) in which the opener member may dwell without opening a blister.

5. A device as claimed in any preceding claim, wherein the mouthpiece (28a) is located at the front of the body, a further air inlet (31) leads into the chamber and wherein a baffle plate (32) is supported by the rear wall (30) of the body and is located inside the chamber.

6. A device as claimed in claim 1, wherein the mouthpiece (35) is located at the front end of the body; the partition (38) has a single locating aperture; and a magazine (39) adapted to contain a plurality of packs (28) is arranged to the rear of the chamber (37).

7. A device as claimed in any preceding claim, wherein said at least one air inlet to the chamber extends through the opener member (12, 24, 42).

8. A device as claimed in any preceding claim, wherein the cover is connected with a captive cap (15, 33) adapted to close the mouthpiece.

9. A device as claimed in claim 1, wherein closure of the cover (7, 26) itself causes the opener member (12, 24) to open the blister (5, 21) located in said aperture (6, 22).

10. A device as claimed in claim 1, wherein closure of the cover (41) moves the opener member (42) into a position such that it can thereafter be moved to open the blister located in said aperture.

**Patentansprüche**

1. Medizinisches Inhalationsgerät, enthaltend einen Körper (1, 1a) mit einer darin angeordneten Kammer (2, 18, 37), die zur Aufnahme eines Behälters (5, 21) für ein Medikament ausgebildet ist und eine Zwischenwand (3, 19, 38) aufweist, auf der der Behälter gehaltert werden kann; mit einem Deckel (7, 26, 41) der normalerweise die Kammer schließt, jedoch geöffnet werden kann, um den Behälter einsetzen und in der Kammer anordnen zu können; mit einem mit der Kammer in Verbindung stehenden Mundstück (9, 28a, 35), durch das ein Patient inhalieren kann; mit mindestens einer in die Kammer führenden Einlaßöffnung (34, 44); und mit mit dem Behälter, während dieser auf der Zwischenwand stationär ist, in Eingriff bringbaren Öffnungseinrichtungen (12, 24, 42), wenn der Deckel geschlossen wird, um es zu ermöglichen, daß Medikament von dem Behälter abgezogen werden kann, wenn der Patient inhaliert, dadurch gekennzeichnet, daß der Behälter ein Blister (5, 21) einer Blisterpackung (4, 20, 28) ist, die auf der Zwischenwand (3, 19, 38) gehaltert wird, um nicht nur während des Öffnens des Blisters sondern auch während des Inhalierens stationär zu bleiben, die Zwischenwand eine sich durch sie erstreckende Anordnungsöffnung (6, 22) aufweist, so daß der Blister darin angeordnet werden kann, der Deckel schwenkbar mit dem Körper verbunden ist und die Öffnungseinrichtungen ein vom dem angelenkten Deckel getragenes Öffnungselement (12, 24, 42) enthalten, das derart positioniert ist, daß das Element, wenn der Deckel geschlossen wird, durch die Anordnungsöffnung (6, 22) in der Zwischenwand hindurchgelangen kann, um dadurch den darin angeordneten Blister zu öffnen.

2. Gerät nach Anspruch 1, worin das Öffnungselement (12, 24) in einer Bahn (13, 25) auf der Innenseite des Deckels gleitbar ist, so daß die Position des Elementes eingestellt werden kann, und die Zwischenwand eine Verweilöffnung (14, 23) aufweist, in der das Element bei geschlossenem Deckel verweilen kann, ohne einen in der Anordnungsöffnung (6, 22) angeordneten Blister zu öffnen.

3. Gerät nach Anspruch 1 oder 2, worin der Deckel (7, 26, 41) derart angeordnet ist, daß er in geschlossenem Zustand benachbart einer Seite der Zwischenwand (3, 19, 38) liegt, und der Lufteinlaß (34, 44) in die Kammer auf der anderen Seite der Zwischenwand führt.

4. Gerät nach Anspruch 1, worin die Zwischenwand (19) zur Halterung einer mit einer Vielzahl von Blistern (21) versehenen Packung (20) ausgebildet ist und eine Vielzahl von Anordnungsöffnungen (22) aufweist, von denen jede zur Aufnahme eines der Blister ausgebildet ist, das Öffnungselement (24) in einer Bahn (25) auf der Innenseite des Deckels bewegbar ist, so daß seine Position derart eingestellt werden kann, daß sie mit der Position einer der Anordnungsöffnungen (22) oder mit der einer Verweilöffnung (23) in der Zwischenwand (19) aufgerichtet ist, in der das Öffnungselement ohne Öffnung eines Blisters verweilen kann.

5. Gerät nach einem vorhergehenden Anspruch, worin das Mundstück (28a) an der Vorderseite des Körpers angeordnet ist, ein weiterer Lufteinlaß (31) in die Kammer führt und worin eine Prallplatte (32) von der hinteren Wand des Körpers gehaltert und innerhalb der Kammer angeordnet ist.

6. Gerät nach Anspruch 1, worin das Mundstück (35) an dem vorderen Ende des Körpers angeordnet ist, die Zwischenwand (38) eine einzige Anordnungsöffnung aufweist und ein zur Aufnahme einer Vielzahl von Packungen (28) ausgebildetes Magazin (39) an der Rückseite der Kammer (37) angeordnet ist.

7. Gerät nach einem vorhergehenden Anspruch, worin der mindestens eine Lufteinlaß in die Kammer sich durch das Öffnungselement (12, 24, 42) erstreckt.

8. Gerät nach einem vorhergehenden Anspruch, worin der Deckel mit einer unverlierbaren Kappe (15, 33) verbunden ist, die zum Verschließen des Mundstücks ausgebildet ist.

9. Gerät nach Anspruch 1, worin das Schließen des Deckels (7, 26) selbst das Öffnungselement (12, 24) veranlaßt, den in der Öffnung (6, 22) angeordneten Blister (5, 21) zu öffnen.

10. Gerät nach Anspruch 1, worin das Schließen des Deckels (41) das Öffnungselement (42) in eine derartige Position bewegt, daß es daran anschließend zum Öffnen des in der Öffnung angeordneten Blisters bewegt werden kann.

## Revendications

1. Un dispositif d'inhalation médical, comprenant un corps (1, 1a) contenant une chambre (2, 18, 37), ladite chambre étant adaptée pour recevoir un récipient (5, 21) pour médicament et comportant une paroi séparatrice (3, 19, 38) zur laquelle le récipient peut être supporté; un couvercle (7, 26, 41) qui ferme normalement la chambre mais qui peut être ouvert pour permettre au récipient d'être introduit et positionné dans la chambre; une pièce d'embouchure (9, 28a, 35) en communication avec la chambre et au travers de laquelle le patient peut inhaler; au moins une entrée d'air (34, 44) aboutissant à la chambre; et un moyen d'ouverture (12, 24, 42) pouvant entrer en contact avec le récipient, quand il est stationnaire sur la paroi séparatrice, lorsque le couvercle est fermé, pour ouvrir le récipient supporté par ladite paroi, afin de permettre au médicament d'être extrait du récipient lorsque le patient inhale, caractérisé en ce que ledit récipient est une ampoule (5, 21) faisant partie d'un paquet d'ampoules (4, 20, 28) qui est supporté par la paroi séparatrice (3, 19, 38) de façon à rester stationnaire non seulement pendant l'ouverture de l'ampoule mais aussi pendant une inhalation, la paroi séparatrice comportant une ouverture de positionnement (6, 22) la traversant de façon que l'ampoule puisse être positionnée dedans; le couvercle est relié de façon articulée avec le corps; et ledit moyen d'ouverture comprend un élément d'ouverture (12, 24, 42) porté par le couvercle articulé et positionné de telle sorte que, lorsque le couvercle est fermé, l'élément puisse passer au travers de l'ouverture de positionnement (6, 22) de la paroi séparatrice pour ouvrir l'ampoule placée dans celle-ci.

2. Un dispositif tel que revendiqué dans la revendication 1, dans lequel l'élément d'ouverture (12, 24) peut glisser dans une voie (13, 25) placée sur le côté intérieur du couvercle de telle sorte que la position de l'élément puisse être réglée; et la paroi séparatrice comporte une ouverture d'attente (14, 23) dans laquelle l'élément peut être en attente, lorsque le couvercle est fermé, sans ouvrir une ampoule située dans l'ouverture de positionnement (6, 22).

3. Un dispositif tel que revendiqué dans la revendication 1 ou la revendication 2, dans lequel le couvercle (7, 26, 41) est agencé, lorsqu'il est fermé, pour être situé adjacent à un côté de la paroi séparatrice (3, 19, 38) et ladite entrée d'air (34, 44) débouche dans la chambre de l'autre côté de la paroi sépatrice.

4. Un dispositif tel que revendiqué dans la revendication 1, dans lequel la paroi séparatrice est adaptée pour supporter un paquet (20) comportant plusieurs ampoules (21) et comporte une pluralité d'ouvertures de positionnement (22) chacune agencée de manière à recevoir une des ampoules, l'élément d'ouverture (24) étant déplaçable dans une voie (25) prévue sur le côté intérieur du couvercle de manière que sa position puisse être réglée en coincidence avec celle de l'une des ouvertures de positionnement (22), ou avec celle d'une ouverture d'attente de la paroi séparatrice (19), dans laquelle l'élément d'ouverture peut attendre sans ouvrir une ampoule.

5. Un dispositif tel que revendiqué dans une quelconque des revendications précédentes, dans lequel la pièce d'embouchure (28a) est située à l'avant du corps, une autre entrée d'air (31) débouche dans la chambre et une plaque formant chicane (32) est supportée par la paroi arrière (30) du corps et est située à l'intérieur de la chambre.

6. Un dispositif tel que revendiqué dans la revendication 1, dans lequel la pièce d'embouchure (35) est située à l'extrémité avant du corps; la paroi séparatrice (38) comporte une seule ouverture de positionnement; et un magasin (39) adapté pour contenir plusieurs paquets (28) est disposé à l'arrière de la chambre (37).

7. Un dispositif tel que revendiqué dans une quelconque des revendications précédentes, dans lequel ladite entrée d'air au moins prévue dans la chambre s'étend au travers de l'élément d'ouverture (12, 24, 42).

8. Un dispositif tel que revendiqué dans une quelconque des revendications précédentes, dans lequel le couvercle est relié à un bouchon captif (15, 33) adapté pour fermer la pièce d'embouchure.

9. Un dispositif tel que revendiqué dans la revendication 1, dans lequel une fermeture du couvercle (7, 26) proprement dit fait en sorte que l'élément d'ouverture (12, 24) ouvre l'ampoule (5, 21) située dans ladite ouverture (6, 22).

10. Un dispositif tel que revendiqué dans la revendication 1, dans lequel une fermeture du couvercle (41) déplace l'élément d'ouverture (42), jusque dans une position de telle sorte qu'il puisse ensuite être déplacé pour ouvrir l'ampoule située dans ladite ouverture.

Fig.1

Fig.2

1

Fig. 3

Fig. 4

Fig. 5

Fig. 6

Fig.7

Fig.8